(19) Europäisches Patentamt
European Patent Office
Office européen des brevets

(11) Numéro de publication: **0 145 620**
**A2**

(12) # DEMANDE DE BREVET EUROPEEN

(21) Numéro de dépôt: **84420193.9**

(22) Date de dépôt: **15.11.84**

(51) Int. Cl.⁴: **C 07 D 213/57**, C 07 D 213/56, C 07 D 213/55, C 07 D 213/68, A 01 N 43/40

(30) Priorité: **24.11.83 FR 8318976**

(43) Date de publication de la demande: **19.06.85 Bulletin 85/25**

(84) Etats contractants désignés: **AT BE CH DE FR GB IT LI LU NL SE**

(71) Demandeur: **RHONE-POULENC AGROCHIMIE, 14-20, rue Pierre Baizet, F-69009 Lyon (FR)**

(72) Inventeur: **Bulot, Jean-Paul, 4 Aallée du Camaïeu, F-69570 Dardilly (FR)**

(74) Mandataire: **Chaumette, Michel et al, RHONE-POULENC AGROCHIMIE BP 9163-09, F-69263 Lyon Cedex 1 (FR)**

(54) **Nouveaux dérivés de l'acide (pyridyl-3) 2 phénylamino-2 acétique, leur préparation et leur utilisation comme antifongiques dans le domaine agricole.**

(57) Nouveaux dérivés de l'acide (pyridyl-3)-2 phénylamino-2 acétique répondant à la formule:

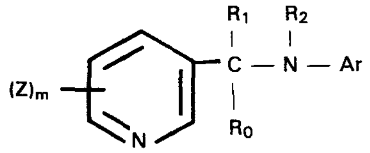

avec:
$R_0$: -CN, -COOH, $CONH_2$, $COOR_4$,
$R_1$: H, alkyle inférieur, cycloalkyle, phényle, éventuellement substitué, aralkyle éventuellement substitué,
$R_2$: H, alkyle inférieur, éventuellement substitué,
Ar: phényle éventuellement substitué,
Z: halogène, alkyle inférieur, alkoxy inférieur, alkylthio inférieur.

Ils sont utilisables en agriculture pour la lutte contre les champignons phytopathogènes tels que les sclérotiniacées.

EP 0 145 620 A2

La présente invention concerne de nouveaux dérivés de l'acide (pyridyl-3)-2 phénylamino-2 acétique, leur préparation et leur utilisation pour la protection des végétaux, contre les champignons phytopathogènes.

Un but de l'invention est la réalisation de produits à usage phytosanitaire présentant une excellente activité vis à vis des champignons phytopathogènes du type sclérotiniacées, notamment le botrytis.

Un autre but de l'invention est la réalisation de fongicides présentant de plus une activité vis à vis d'autres champignons phytopathogènes, responsables des maladies des cultures, tels que les maladies fongiques des céréales (piétain-verse, septorioses, oïdium, rouilles), les cercosporioses et les maladies des arbres fruitiers.

Il a maintenant été trouvé que ces buts pouvaient être atteints grâce aux produits de l'invention.

Ceux ci sont caractérisés en ce qu'ils répondent à la formule générale (I).

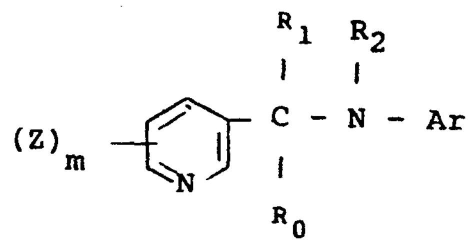

(I)

dans laquelle

$R_0$ représente un radical choisi parmi les radicaux -CN, -COOH, $-CONH_2$ et le radical $-COOR_4$ dans lequel $R_4$ représente un radical alkyle inférieur

$R_1$ représente l'atome d'hydrogène ou un radical alkyle inférieur, éventuellement substitué, (par exemple par un ou plusieurs substituants choisis parmi les atomes d'halogènes et les radicaux alkoxy inférieur et alkylthio inférieur) ou un radical cycloalkyle, comportant de 3 à 7 chaînons (par exemple cyclopropyle, cyclobutyle, cyclopentyle, cyclohexyle) lui-même éventuellement substitué ou

un radical phényle lui-même éventuellement substitué (par exemple par un ou plusieurs atomes d'halogènes ou radicaux alkyle inférieur) ou aralkyle éventuellement substitué (par exemple benzyle ou phénéthyle éventuellement substitué par 1 ou plusieurs atomes d'halogènes).

$R_2$ représente l'atome d'hydrogène ou un radical alkyle inférieur éventuellement substitué (par exemple par un ou plusieurs halogènes, alkoxy ou alkylthio inférieurs), sous réserve que $R_1$ et $R_2$ ne représentent pas simultanément l'atome d'hydrogène,

m est un nombre entier de 0 à 4, limites comprises, étant entendu que lorsque m est supérieur à 1, les substituants Z peuvent être soit identiques, soit différents.

Ar représente un radical phényle éventuellement substitué, par exemple par un ou plusieurs substituants choisis parmi les atomes d'halogènes, les radicaux alkyle inférieur et cycloalkyle inférieur eux-mêmes éventuellement substitués, (par exemple par un ou plusieurs atomes d'halogène), les radicaux alkoxy inférieur eux-mêmes éventuellement substitués (par exemple par un ou plusieurs atomes d'halogènes) ; alkylthio inférieur eux-mêmes éventuellement substitués (par exemple par un ou plusieurs atomes d'halogènes) ; cyano ; nitro ; carboxy ; alkoxycarbonyle inférieur ; hydroxyle et le radical phényle lui-même éventuellement substitué) .

Au sens du présent texte, l'adjectif "inférieur", lorsqu'il qualifie un radical organique signifie que ce radical comporte au plus six atomes de carbone. Ce radical peut être soit linéaire, soit ramifié.

Les composés selon la formule (I) pour lesquels $R_0$ représente un groupe $-CN$ ou $-COOR_4$ ou $-CONH_2$

peuvent former divers sels d'addition avec des acides appropriés pouvant être minéraux, tels que par exemple l'acide chlorhydrique, l'acide sulfurique, l'acide phosphorique, ou organiques tels que par exemple l'acide succinique, l'acide fumarique, l'acide maléique, l'acide oxalique ou l'acide tartrique. De plus, les composés selon la formule (I) pour lesquels $R_0$ représentent un groupe -COOH peuvent donner des sels avec des bases appropriées. Ces divers sels sont compris dans le cadre de la présente invention tout particulièrement lorsqu'ils sont acceptables en agriculture (c'est-à-dire acceptables pour les plantes traitées).

Ils peuvent être préparés selon des méthodes en soi connues, par exemple en dissolvant le composé (I) dans un solvant approprié, puis en faisant agir un acide ou une base (selon la signification de $R_0$) appropriés.

Les composés selon la formule (I) comportent tous, un atome de carbone substitué de façon asymétrique situé en position alpha par rapport au groupe pyridyle. De ce fait chacun des composés selon la formule (I) peut exister sous plusieurs formes stéréoisomères, dont les niveaux d'activité antifongique peuvent différer entre eux. Ces formes stéréoisomères ainsi que leurs mélanges, optiquement actifs ou racémiques, sont également inclus dans le cadre de la présente invention.

Divers dérivés hétérocycliques de l'acétonitrile ont déjà été décrits :

Ainsi, la demande de brevet européen EP-A 48 039 décrit des dérivés du phénylamino-2 acétonitrile substitué en position 2 de l'acétonitrile par un radical hétérocyclique comportant nécessairement cinq chaînons. D'après cette référence, ces composés sont utilisables comme régulateurs de la croissance des plantes.

La demande de brevet européen EP-A 8 145 et son équivalent américain US-A-4 281 134 décrivent en tant que régulateurs de la croissance des plantes et fongicides

divers dérivés de phényliminopyridine. La revue Chem. Ind. (London) 1968 (5) p.155 (Chemical Abstract 68 n°68 602 g) décrit des dérivés du phénylamino-2 acétonitrile substitués en position 2 sur l'acétonitrile par un radical pyridyl-2, furyl-2 ou thienyl-2 (à l'exclusion de tout radical pyridyl-3). Aucune utilisation n'est mentionnée pour ces composés.

La demande de brevet allemand DE-A 1 026 318 décrit plusieurs dialkylamino-2 (pyridyl-2)-3 acétonitriles utilisables comme médicaments.

Par ailleurs enfin, le brevet américain US-A- 3 313 683 mentionne, d'une façon générale, une famille très vaste de dérivés de l'acétonitrile, comprenant des arylamino-2 aryl-2 acétonitriles, des alkylamino-2 aryl-2 acétronitriles et des dialkylamino-2 aryl-2 acétonitriles. Il ne décrit toutefois qu'un seul dérivé de pyridyl-3)-2 acétonitrile, différent de ceux revendiqués par la présente demande de brevet et indique que ces composés sont actifs à la fois comme nématicides et comme fongicides actif contre les champignons du sol tels que Rhizoctonia solani, Fusarium oxysporum, Sclerotium rolfsii

Les composés selon l'invention sont différents de ceux décrits dans les divers documents mentionnés plus haut. Leurs excellentes propriétés antifongiques, notamment à l'encontre du Botrytis n'étaient pas prévisibles compte tenu de cet art antérieur.

Parmi les composés selon la formule (I), l'invention concerne plus spécialement les composés répondant à la formule (II) :

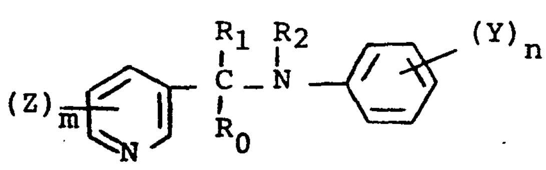

(II)

et les sels acceptables en agriculture de ces composés (II).

Dans la formule (II) :

$R_0$ représente le groupe -CN ou le groupe $-COOR_4$ dans lequel $R_4$ représente un radical alkyle inférieur

$R_1$ représente un radical alkyle inférieur ou cycloalkyle inférieur,

$R_2$ représente l'atome d'hydrogène ou le radical méthyle,

m est un entier égal à 0,1 ou 2

Z représente un radical alkyle ou alkoxy, comportant de 1 à 3 atomes de carbone, étant entendu que, lorsque m est égal à 2 les substituants Z peuvent être identiques ou différents

n est un entier égal à 0,1, 2, 3 ou 4

Y représente un atome d'halogène (par exemple fluor, chlore, brome) ou un radical alkyle comportant de 1 à 3 atomes de carbone (par exemple méthyle), halogénoalkyle comportant de 1 à 3 atomes de carbone (par exemple trifluorométhyle), alkoxy comportant de 1 à 3 atomes de carbone (par exemple méthoxy), alkylthio comportant de 1 à 3 atomes de carbone (par exemple méthylthio), alkoxycarbonyle inférieur, phényle, ou cycloalkyle inférieur (par exemple cyclohexyle).

Parmi les composés répondant à la formule (II), une sous-famille préférée du fait de son efficacité antifongique vis à vis du Botrytis est constituée par les composés pour lesquels :

$R_0$ a la même signification que dans la formule (II)

$R_1$ représente un radical alkyle comportant de 1 à 5 atomes de carbone,

$R_2$ représente l'atome d'hydrogène,

n est égal à 1 ou à 2

Y représente un atome de fluor, chlore ou brome étant entendu que lorsque n est égal à 2, les atomes Y peuvent être identiques ou différents.

m est égal à zéro, 1 ou 2

Z représente un radical méthyle ou éthyle, ou méthoxy.

De préférence l'un des atomes d'halogène Y est en position 4 sur le noyau phényle.

La présente invention concerne de plus la préparation des composés répondant à la formule (I).

Les composés selon la formule (I) pour lesquels $R_0$ représente le groupe -CN peuvent être obtenus selon un premier procédé en faisant réagir le dérivé de pyridine de formule (III) :

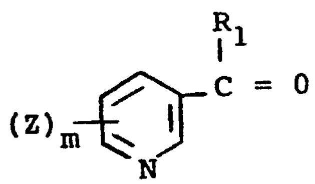

(III)

dans laquelle $R_1$ Z et m ont la même signification que dans la formule I,

- sur le dérivé d'aniline de formule (IV) :

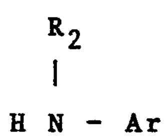

(IV)

dans laquelle Ar et $R_2$ ont la même signification que dans la formule (I),

- en opèrant en présence d'acide cyanhydrique, éventuellement formé "in situ".

La réaction s'effectue avantageusement à une température de l'ordre de 0 à 100°C, éventuellement en présence d'un solvant, inerte, (c'est-à-dire ne réagissant pas avec les réactifs en présence dans les conditions de la réaction). Ce solvant peut être choisi avantageusement parmi les hydrocarbures, aromatiques ou aliphatiques ou cycloaliphatiques, ces hydrocarbures étant éventuellement halogènés, tels que, par exemple le toluène, les xylènes, le chlorobenzène, les dichlorobenzènes etc... D'autres

solvants inertes peuvent également être utilisés sans sortir du cadre de l'invention.

Selon un mode de réalisation de l'invention, l'acide cyanhydrique est ajouté au milieu réactionnel, sous la forme d'une solution, ou sous forme gazeuse, ou sous forme liquide en opérant sous pression.

De préférence l'acide cyanhydrique est formé "in situ" dans le milieu réactionnel, en faisant agir un acide approprié sur un cyanure de métal alcalin. L'acide peut être un acide minéral, tel que par exemple l'acide chlorhydrique, ou un acide organique, tel que par exemple l'acide acétique, qui peut alors être utilisé à la fois comme réactif et comme solvant.

Avantageusement, cette formation in situ est obtenue par action de l'acide acétique sur une solution aqueuse de cyanure de potassium.

Les composés selon la formule (I) pour lesquels $R_0$ représente le groupe -CN peuvent également être obtenus selon un deuxième procédé.

Ce procédé est caractérisé en ce qu'il consiste :

- en une première étape à faire réagir le dérivé de pyridine de formule (III)

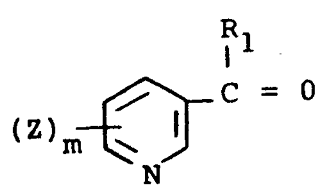

(III)

dans laquelle $R_1$ Z et m ont la même signification que dans la formule (I),

-sur le dérivé d'aniline de formule (V)

$$H_2\ N-Ar \qquad (V)$$

dans laquelle Ar a la même signification que dans la formule (I),

- pour donner le dérivé de phényliminométhyl-3 pyridine de formule (VI)

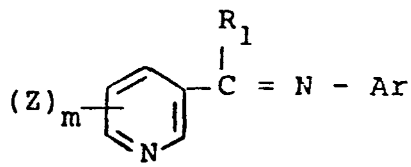

(VI)

dans laquelle $R_1$, Z, m et Ar ont la même signification que dans la formule (I),

- puis en une deuxième étape à faire réagir l'acide cyanhydrique éventuellement formé in situ, sur ce dérivé de phényliminométhyl-3 pyridine de formule (VI).

Avantageusement la première étape de ce procédé s'effectue à une température de l'ordre de 20°C à 150°C dans un solvant inerte (c'est-à-dire ne réagissant pas vis à vis des réactifs en présence), en présence d'un agent de déshydratation usuel, tel qu'un acide fort (par exemple l'acide sulfurique, ou l'acide p-toluène sulfonique) ou un anhydride ou chlorure d'acide (par exemple $P_2O_5$ ou $POCl_3$) ou encore un acide au sens de Lewis. De préférence l'eau est éliminée du milieu réactionnel par distillation azéotropique.

Comme solvant utilisable pour cette première étape, on peut mentionner des hydrocarbures aromatiques, ou aliphatiques, ou cycloaliphatiques, ces hydrocarbures étant eux-mêmes éventuellement halogénés, tels que par exemple le toluène, les xylènes, le chlorobenzène, les dichlorobenzènes, ou encore des nitriles tel que l'acétonitrile, ou encore des amides tel que le N,N-diméthylformamide, ou encore des alcanols tel que l'éthanol, cette liste n'étant pas limitative. On opère avantageusement à la température d'ébullition du solvant utilisé.

La deuxième étape du procédé s'effectue avantageusement à une température de l'ordre de 0 à 100°C environ, éventuellement en présence d'un solvant inerte. Ce

solvant est généralement choisi parmi ceux mentionnés ci-dessus en ce qui concerne la première étape du procédé.

Selon un mode particulier de réalisation de l'invention, l'acide cyanhydrique est ajouté au milieu réactionnel, sous la forme d'une solution, ou sous forme liquide, ou sous forme gazeuse.

De préférence toutefois, l'acide cyanhydrique est formé "in situ" dans le milieu réactionnel, en opèrant selon la méthode déjà décrite dans le cas du premier procédé.

Les composés selon la formule (I) pour lesquels $R_0$ représente le groupe $-CONH_2$ peuvent être préparés par hydratation des nitriles correspondants en opérant selon toute méthode usuelle pour ce type de réaction et adaptée à cette transformation. On peut aussi faire agir un acide fort tel que HCl dans le méthanol, puis l'hydrolyser.

Les composés selon la formule (I) pour lesquels $R_0$ représente le groupe -COOH peuvent être préparés à partir des amides décrites plus haut par hydrolyse en milieu acide conduisant à un chlorhydrate de l'acide que l'on neutralise ensuite par une base pour obtenir l'acide.

Les composés selon la formule (I) pour lesquels $R_0$ représente un groupe $-COOR_4$, dans lequel $R_4$ représente un radical alkyle inférieur peuvent être obtenus par estérification del'acide obtenu précedemment au moyen d'un alcool de formule $R_4OH$.

En fin de réaction, quelque soit le procédé utilisé, le composé formé est isolé du milieu réactionnel par les moyens usuels, tel que, par exemple, par distillation du solvant, ou par cristallisation du produ dans le milieu réactionnel, ou par filtration, puis, si nécessaire, il est ensuite purifié, par exemple par recristallisation dans un solvant approprié, ou par chromatographie.

Les exemples ci-après, décrits à titre non limitatif, illustrent la préparation des composés selon

l'invention, ainsi que leur utilisation pour la lutte contre les champignons phytopathogènes. Les structures des composés décrits dans ces exemples ont été confirmées par spectrométrie de résonance magnétique nucléaire (R.M.N.) et/ou par spectrométrie infra-rouge.

Exemple 1 : Préparation du phénylamino-2(pyridyl-3)-2 propanenitrile (composé n° 1), de formule :

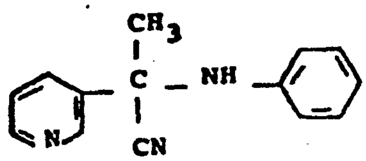

On utilise un ballon tricol surmonté d'un agitateur et équipé d'un thermomètre et d'un réfrigérant ascendant. Dans le ballon, on charge successivement 50 ml d'acide acétique, 12,1 g d'acétyl-3 pyridine et 9,3 g d'aniline, et on maintient le mélange sous agitation, pendant 30 minutes, à température ambiante.

On ajoute alors 8,5 g de cyanure de potassium en solution dans 20 ml d'eau.

Le mélange est maintenu pendant 20 heures à température ambiante et le précipité formé est séparé par filtration. Le solide ainsi obtenu est lavé successivement par de l'eau (200 ml), par 100 ml d'une solution aqueuse, à 10 %, de $KHCO_3$, par de l'eau (200 ml) puis par du pentane.

Le solide brut obtenu est recristallisé dans 30 g d'éthanol à reflux. On obtient 14 g (0,063 mole) de phénylamino-2(pyridyl-3)-2 propanenitrile sous la forme d'un solide blanc fondant à 156°C.
Rendement (calculé à partir de l'acétylpyridine) : 63 %.

Exemple 2 : Préparation du (phényl-4 phényl) amino-2(pyridyl-3)-2 propanenitrile (composé n° 2), de formule :

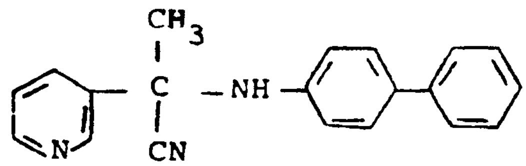

Cette préparation s'effectue selon deux étape successives en utilisant le dispositif décrit dans l'exemple 1.

6 g d'acétyl-3 pyridine, 9,3 g d'amino-4 biphényle et 0,9 g d'acide p-toluène sulfonique sont dissous dans 100 ml de toluène. La solution est chauffée à la température d'ébullition du solvant pendant 4 heures et l'eau formée est recueillie dans un récepteur de Dean-Stark.

Après refroidissement, la solution est lavée par l'eau (3 x 100 ml), séchée sur sulfate de sodium, en présence de noir animal puis filtrée et évaporée sous pression réduite.

On obtient ainsi 11,3 g de [(phényl-4 phényl) imino-1]éthyl-3 pyridine de formule :

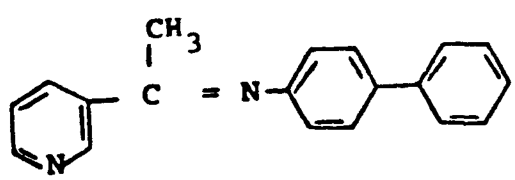

A 11,3 g de [(phényl-4 phényl) imino-1]éthyl-3 pyridine dans 50 ml d'acide acétique, on ajoute 3,9 g de cyanure de potassium. Le mélange est agité pendant 24 heures à température ambiante. Par filtration du mélange réactionnel on sépare un solide blanc que l'on lave successivement par 300 ml d'eau, par 300 ml d'une solution à 10 % de carbonate acide de potassium, et par 300 ml d'eau. Après séchage du produit sous pression réduite, on obtient 7,8 g de (phényl-4 phényl) amino-2 (pyridyl-3)-2 propanenitrile, sous la forme d'un solide blanc fondant à 189°C.

Rendement (calculé à partir de l'acétyl-3 pyridine) : 48 %.

Exemple 3 :

En opérant selon l'une ou l'autre des méthodes décrites dans les exemples 1 et 2, les composés mentionnés ci-après ont été préparés :

La méthode utilisée a été celle de l'exemple 1 pour les composés n° 3 à 7, et celle de l'exemple 2 pour les composés n° 8 à 63 et 72.

- [(méthyl) (chloro-4 phényl)amino]-2(pyridyl-3)-2 acétonitrile, (composé n° 3),
- (chloro-4 phényl)amino-2(pyridyl-3)-2 propanenitrile, (composé n° 4),
- (fluoro-4 phényl)amino-2(pyridyl-3)-2 propanenitrile, (composé n°5),
- (fluoro-2 phényl)amino-2(pyridyl-3)-2 propanenitrile, (composé n° 6),
- (bromo-4 phényl)amino-2(pyridyl-3)-2 propanenitrile, (composé n° 7),
- (dichloro-3,4 phényl)amino-2(pyridyl-3)-2 propanenitrile, (composé n° 8),
- (chloro-3 phényl)amino-2(pyridyl-3)-2 propanenitrile, (composé n° 9),
- (dichloro-2,4 phényl)amino-2(pyridyl-3)-2 propanenitrile, (composé n° 10),
- (fluoro-4 phényl)amino-2 phényl-2(pyridyl-3)-2 acétonitrile, (composé n° 11),
- phénylamino-2 phényl-2(pyridyl-3)-2 acétonitrile, (composé n° 12),
- (difluoro-2,4 phényl)amino-2(pyridyl-3)-2 propanenitrile, (composé n° 13),
- (fluoro-3 phényl)amino-2(pyridyl-3)-2 propanenitrile, (composé n° 14),
- (chloro-3 fluoro-4 phényl)amino-2(pyridyl-3)-2 propanenitrile (composé n° 15),
- (cyano-3 phényl)amino-2 (pyridyl-3)-2 propanenitrile, (composé n° 16),

- (bromo-3 phényl)amino-2 (pyridyl-3)-2 propanenitrile, (composé n° 17),
- (méthyl-3 phényl)amino-2 (pyridyl-3)-2 propanenitrile, (composé n° 18),
- (chloro-4 phényl)amino-2 (pyridyl-3)-2 pentanenitrile, (composé n° 19),
- (bromo-3 chloro-4 phényl)amino-2 (pyridyl-3)-2 propanenitrile, (composé n° 20),
- (dichloro-3,4 phényl)amino-2 (pyridyl-3)-2 pentanenitrile, (composé n° 21),
- (trichloro 3,4,5 phényl)amino-2 (pyridyl-3)-2 propanenitrile, (composé n° 22),
- (chloro-3 fluoro-4 phényl)amino-2 (pyridyl-3)-2 pentanenitrile, (composé n° 23),
- (dichloro 3,4 phényl)amino-2 (pyridyl-3)-2 phényl-2 acétonitrile, (composé n° 24),
- (chloro-4 phényl)amino-2 (pyridyl-3)-2 méthyl-4 pentanenitrile, (composé n° 25),
- (dichloro-3,4 phényl)amino-2 (pyridyl-3)-2 méthyl-4 pentanenitrile, (composé n° 26),
- (bromo-3 chloro-4 phényl)amino-2 (pyridyl-3)-2 phényl-2 acétonitrile, (composé n° 27),
- (chloro-3 fluoro-4 phényl)amino-2 (pyridyl-3)-2 méthyl-4 pentanenitrile, (composé n° 28),
- (methoxycarbonyl-4 phényl)amino-2 (pyridyl-3)-2 propanenitrile, (composé n° 29),
- (fluoro-4 phényl)amino-2 (pyridyl-3)-2 pentanenitrile, (composé n° 30),
- (méthylthio-3 phényl)amino-2 (pyridyl-3)-2 propanenitrile, (composé n° 31),
- (chloro-4 phényl)amino-2 (pyridyl-3)-2 butanenitrile (composé n° 32),
- (chloro-3 fluoro-4 phényl)amino-2 (pyridyl-3)-2 butanenitrile (composé n°33),
- (chloro-4 phényl)amino-2 (pyridyl-3)-2 hexanenitrile (composé n° 34),

- (dichloro-3,4 phényl)amino-2 (pyridyl-3)-2 butanenitrile, (composé n° 35),
- (bromo-3 phényl)amino-2 (pyridyl-3)-2 butanenitrile, (composé n° 36),
- (chloro-4 phényl)amino-2 (pyridyl-3)-2 méthyl-3 butanenitrile (composé n° 37),
- (fluoro-4 phényl)amino-2 (pyridyl-3)-2 méthyl-3 butanenitrile, (composé n° 38),
- (fluoro-4 phényl)amino-2 (pyridyl-3)-2 butanenitrile, (composé n° 39),
- (chloro-3 fluoro-4 phényl)amino-2 (pyridyl-3)-2 phényl-3 propanenitrile (composé n°40),
- (trifluorométhyl-4 phényl)amino-2 (pyridyl-3)-2 butanenitrile, (composé n° 41),
- (difluoro-3,4 phényl)amino-2 (pyridyl-3)-2 butanenitrile, (composé n° 42),
- (phénylamino-2 (pyridyl-3)-2 butanenitrile, (composé n° 43),
- (chloro-3 fluoro-4 phényl)amino-2 (pyridyl-3)-2 méthylthio-3 propanenitrile (composé n°44),
- (bromo-4 phényl)amino-2 (pyridyl-3)-2 pentanenitrile, (composé n° 45),
- (bromo-4 phényl)amino-2 (pyridyl-3)-2 butanenitrile, (composé n° 46),
- (chloro-4 phényl)amino-2 (pyridyl-3)-2 cyclopentyl-2 éthanenitrile (composé n° 47),
- (difluoro-3,4 phényl)amino-2 (pyridyl-3)-2 pentanenitrile, (composé n° 48),
- (difluoro-3,4 phényl)amino-2 (pyridyl-3)-2 méthyl-3 butanenitrile, (composé n° 49),
- (trifluorométhyl-4 phényl)amino-2 (pyridyl-3)-2 méthyl-3 butanenitrile, (composé n° 50),
- (chloro-4 phényl)amino-2 (méthyl-5 pyridyl-3)-2 butanenitrile (composé n° 51),
- (chloro-4 phényl)amino-2 (méthyl-4 pyridyl-3)-2 butanenitrile (composé n° 52),

- (chloro-3 fluoro-4 phényl)amino-2 (méthyl-4 pyridyl-3)-2 butanenitrile (composé n° 53),
- (chloro-4 phényl)amino-2 (diméthyl-4,5 pyridyl-3)-2 butanenitrile (composé n° 54),
- (chloro-3 fluoro-4 phényl)amino-2 (éthyl-4 pyridyl-3)-2 butanenitrile (composé n° 55),
- (fluoro-4 phényl)amino-2 (éthyl-4 pyridyl-3)-2 butanenitrile (composé n° 56),
- (dichloro-3,4 phényl)amino-2 (méthyl-4 pyridyl-3)-2 propanenitrile (composé n° 57),
- (chloro-4 phényl)amino-2 (méthyl-4 pyridyl-3)-2 propanenitrile (composé n° 58),
- (chloro-4 phényl)amino-2 (méthoxy-4 pyridyl-3)-2 butanenitrile (composé n° 59),
- (chloro-4 phényl)amino-2 (méthoxy-4 pyridyl-3)-2 propanenitrile (composé n° 60),
- (dichloro-3,4 phényl)amino-2 (méthoxy-4 pyridyl-3)-2 propanenitrile (composé n° 61),
- (difluoro-3,4 phényl)amino-2 (méthyl-4 pyridyl-3)-2 butanenitrile (composé n° 62),
- (bromo-4 phényl)amino-2 (méthyl-4 pyridyl-3)-2 butanenitrile (composé n° 63),
- (fluoro-4 phényl)amino-2 (isopropyl-4 pyridyl-3)-2 butanenitrile (composé n° 72),

<u>Exemple 4</u> : <u>Préparation du chlorhydrate du composé n° 4</u>

A une solution de 5 g de (chloro-4 phényl)amino-2 (pyridyl-3)-2 propanenitrile, dans 2,5 l d'éther anhydre, on ajoute 18 ml d'une solution d'HCl à 1,08 N, dans l'éther anhydre. On filtre et on sèche et on obtient ainsi 5,5 g du chlorhydrate du composé n° 4, fondant à 120°C.

La formule des composés cités plus haut, leur point de fusion ainsi que les rendements obtenus dans leur préparation sont indiqués dans le tableau I à la fin de la description.

Exemple 5 : Préparation de la (dichloro-3,4 phényl)amino-2 (pyridyl-3)-2 propanamide (composé n° 64)

Dans un ballon tricol, on charge 20 g de (dichloro-3,4 phényl)amino-2 (pyridyl-3)-2 propanenitrile (composé n°8) et 170 ml de méthanol anhydre.

Arpès refroidissement de la suspension à 0,5°C, on fait barboter lentement un courant d'acide chlorydrique gazeux jusqu'à saturation. Après retour à la température ambiante on agite pendant 16 heures puis on dilue le mélange réactionnel avec 150 ml d'éther anhydre ; le solide obtenu est essoré rapidement, lavé avec 300 ml d'éther anhydre puis mis dans une solution aqueuse à 10 % d'hydrogénocarbonate de potassium. Après filtration, lavage à l'eau distillée à l'héthanol et sèchage sous vide on obtient 19,6 g de (dichloro-3,4 phényl)amino-2 propanamide sous forme d'un solide blanc fondant à 175°C (rendement 92 %).

Exemple 6 : Préparation de l'acide (dichloro-3,4 phényl)amino-2 (pyridyl-3)-2 propanoïque (composé n°65)

Dans un ballon tricol, on charge 19,2 g de (dichloro-3,4 phényl)amino-2 (pyridyl-3)-2 propanamide (composé n°64) et 190 ml d'acide chlorydrique-6N, puis on chauffe à reflux pendant 7 heures. Il se forme alors un précipité que l'on filtre et que l'on traite avec 800 ml d'eau et 25 ml d'ammoniaque concentrée.

La solution de sel d'ammonium chauffée à 50°C est ensuite filtrée puis traitée avec 25 ml d'acide acétique glacial.

Après filtration, le solide est lavé à l'eau puis sèché sous vide. On obtient 13,2 g d'acide (dichloro-3,4 phényl)amino-2 (pyridyl-3)-2 propanoïque, sous forme d'un solide fondant à 190°C.

Exemple 7 : Préparation du (dichloro-3,4 phényl)amino-2 (pyridyl-3)-2 propanoate de méthyle (composé n°66)

Dans un ballon tricol, on charge 4,8 g d'acide (dichloro-3,4 phényl)amino-2 (pyridyl-3)-2 propanoïque (composé n°64) dans 25 ml de méthanol anhydre. Après refroidissement à moins 10°C, on ajoute en une fois 1,3 ml de chlorure de sulfinyle, puis on porte lentement au reflux.

Au bout de 3 heures, on refroidit de nouveau à moins 10°C, puis on ajoute encore 1,3 ml de chlorure de sulfinyle.

Après 5 heures de chauffage à reflux on évapore le mélange réactionnel sous pression réduite puis on traite le produit brut successivement avec 50 ml d'éther, 50 ml d'eau distillée, 5 g d'hydrogénocarbonate de potassium.

Après décantation, la phase organique est sèchée sur sulfate de sodium puis évaporée. Le résidu huileux est purifié par chromatographie sur colonne de silice (éluant : éther). On obtient 3,5 g de (dichloro-3,4 phényl)amino-2 (pyridyl-3)-2 propanoate de méthyle, de couleur jaune pâle. Rendement 72 %.

Exemple 8 :

En opérant selon la méthode décrite dans l'exemple 7 précédant à partir des matières de départ appropriées, les composés ci-après ont été préparés

n°67 : (chloro-4 phényl)amino-2 (pyridyl-3)-2 propanoate de méthyle, fondant à 94°C.

n°68 : (chloro-4 phényl)amino-2 (pyridyl-3)-2 butanoate de méthyle, fondant à 154°C.

n°69 : (dichloro-3,4 phényl)amino-2 (pyridyl-3)-2 butanoate de méthyle, fondant à 112°C.

n°70 : (bromo-3 chloro-4 phényl)amino-2 (pyridyl-3)-2 butanoate de méthyle, fondant à 110°C.

n°71 : (dichloro-3,4 phényl)amino-2 (pyridyl-3)-2 bentanoate de méthyle, sous la forme d'une huile jaune.

Les exemples ci-après n°9 à 11 illustrent l'activité biologique des composés selon l'invention.

Exemple 9 : Essai de serre sur l'oïdium de l'orge -

<u>traitement curatif</u>

On prépare par broyage fin une suspension aqueuse de la matière active à tester ayant la composition suivante :

-matière active à tester........................ 40 mg

-Tween 80 (agent tensioactif constitué d'un monolaurate de dérivé polyoxyéthyléné du sorbitan) ............ 0,4 ml

-eau ........................................... 40 ml

Cette suspension aqueuse est ensuite diluée par de l'eau pour obtenir la concentration désirée.

Des plants d'orge (*Hordeum vulgare*), de variété Bérac, sont cultivés en godets. Lorsque ces plants sont agés de cinq jours ils sont contaminés par saupoudrage au moyen de spores d'oïdium de l'orge (*Erysiphe graminis*) puis maintenus en serre à 22°C plus ou moins 2°C sous 70 à 80 % d'humidité relative.

Deux jours après la contamination, alors que le feuillage est envahi par l'oïdium les plants sont traités en pulvérisant sur chacun d'eux 8 ml d'une suspension de la matière active dans de l'eau distillée contenant 0,02 % en poids de Tween 80, à la concentration désirée.

Chaque concentration de la matière active fait l'objet de trois répétitions. Les plants témoins sont traités de la même façon mais sans matière active.

Les plants traités sont ensuite placés de nouveau en serre, dans les conditions de température et humidité décrites plus haut.

Dix jours après le traitement par la suspension de la matière active, on évalue le pourcentage d'inhibition du développement du champignon, par comparaison avec le témoin non traité.

Dans ces conditions, on a observé que, à la concentration de 500 mg/l, le pourcentage d'inhibition du développement du champignon était respectivement :

- d'au moins 90 % pour les composés n°4, 10, 15, 17, 19, 20, 21, 25, 26, 27, 28, 30, 32, 33, 34, 35, 36, 37, 38, 39,

42, 43,

- d'au moins 70 % (mais inférieur à 90 %) pour les composés n°1, 2, 3, 5, 6, 7, 8, 9, 11, 12, 13, 14, 18, 23, 29, 40, 44, 51, 56,
- d'au moins 20 %, mais inférieur à 70 % pour les composés n°41, 47, 48.

Exemple 10 : Activité antifongique sur Botrytis cinerea de la tomate :

Des tomates cultivées en serre (variété Marmande) agées de 60 à 75 jours sont traitées par pulvérisation avec des suspensions aqueuses de même composition que celle décrite à l'exemple précédent et à diverses concentrations de matière active. L'essai est répété deux fois avec chaque concentration.

Après 24 heures, les feuilles sont coupées et mises dans 2 boites Pétri (diamètre 11 cm) dont le fond a été préalablement garni d'un disque de papier filtre humide (5 folioles par boite).

L'inoculum est ensuite apporté à l'aide d'une seringue par dépôt de gouttes (3 gouttes par foliole) d'une suspension de spores. Cette suspension de spores de Botrytis cinerea a été obtenue à partir d'une culture de 15 jours, mise ensuite en suspension dans une solution nutritive (80.000 unités/$cm^3$). Le contrôle est fait environ 4 jours après la contamination par comparaison avec un témoin non traité. On évalue ainsi le pourcentage de protection par rapport au témoin non traité.

Dans ces conditions, on observe que, à la concentration de 1 000 mg/l, le pourcentage de protection était respectivement

- d'au moins 95 % pour les composés n°4, 5, 7, 8, 9, 14, 15, 19, 23, 29, 30, 32, 33, 34, 35, 37, 42, 43, 44, 45, 48, 49, 55, 56, 57, 58, 59, 61, 62, 63, 66
- d'au moins 80 % (mais inférieur à 95 %) pour les composés n°17, 25, 28, 51, 60,
- d'au moins 50 % (mais inférieur à 80 %) pour les composés

n°16, 18, 20, 36, 38, 39, 41, 46, 50, 52, 65,
- d'au moins 20 %, (mais inférieur à 50 %) pour les composés n°24, 26, 40, 53 et 54.

Exemple 11 : Activité antifongique sur la cercosporiose de la betterave (Cercospora Beticola)

Des betteraves sucrières (Beta vulgaris) variété Monostar sont cultivées en serre dans des godets de matière plastique contenant un mélange sable/tourbe de rivière (1/1). Après 12 jours, lorsque les plantules sont au stade 2 feuilles, hauteur 5 à 7 cm, elles sont traitées par pulvérisation avec des suspensions aqueuses de même composition que celle décrite dans l'exemple précédent, à diverses concentrations de matière active. Chaque godet reçoit environ 5 ml de préparation, correspondant sensiblement à une dose de 1 000 l/ha. A chaque concentration de matière active correspondent trois godets.

L'inoculum est ensuite apporté à l'aide d'un pulvérisateur, de telle sorte que chaque godet reçoive environ 5 ml de cette suspension. Après cette contamination, les plantes sont mises en incubation, d'abord pendant 2 jours à 26°C, en atmosphère saturée d'humidité et à l'obscurité ensuite durant 12 jours environ dans les conditions normales de serre (22 + 2°C et 60 à 80 % d'humidité).

Le contrôle est fait quatorze jours après la contamination et on évalue ainsi le pourcentage de protection par rapport au témoin non traité.

Dans ces conditions, on observe que, à la concentration de 1 000 mg/l, le pourcentage de protection était respectivement
- d'au moins 95 % pour les composés n°19, 21, 23, 25, 26, 28, 30, 32, 33, 37, 42,
- d'au moins 80 % (mais inférieur à 95 %) pour les composés n°35, 36, 46,
- d'au moins 50 % (mais inférieur à 80 %) pour les composés n°20, 29.

Ces résultats illustrent la bonne activité antifongique des composés selon l'invention .

Les composés selon l'invention présentent un grand intérêt par leur activité sur sclérotiniacées, notamment sur Botytris et de ce fait peuvent être appliqués sur des cultures telles que les céréales (par exemple l'orge), le riz, la vigne, la betterave à sucre, certaines cultures tropicales telles que arachide et bananier, les cultures maraîchères et florales et en arboriculture (arbres fruitiers). Parmi les composés décrits plus haut, les composés n°4, 7, 8, 19, 21, 23, 32, 33, 35, 37, 42, 44, 55 sont particulièrement préférés.

Pour leur emploi pratique, les composés selon l'invention sont rarement utilisés seuls. Le plus souvent ces composés font partie de compositions. Ces compositions, utilisables comme agents antifongiques, contiennent comme matière active un composé selon l'invention tel que décrit précédemment en association avec les supports solides ou liquides, acceptables en agriculture et les agents tensioactifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment fongicides ). Plus généralement les composés utilisés dans l'invention peuvent être associés à tous les additifs solides ou liquides correspondant aux techniques habituelles de la mise en formulation.

D'une façon générale, les compositions selon l'invention contiennent habituellement de 0,001 à 95 % environ (en poids) d'une ou plusieurs matières actives

selon l'invention, de 1 % à 95 % environ de un ou plusieurs supports solides ou liquides et éventuellement de 0,1 à 20% environ de un ou plusieurs agents tensioactifs.

Selon ce qui a déjà été dit les composés utilisés dans l'invention sont généralement associés à des supports et éventuellement des agents tensioactifs.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau ; alcools, notamment le butanol ; esters, notamment l'acétate de méthyl-glycol ; cétones, notamment la cyclohexanone et l'isophorone ; fractions de pétrole ; hydrocarbures aromatiques, notamment les xylènes, ou paraffiniques ; hydrocarbures chlorés aliphatiques, notamment le trichloroéthane, ou aromatiques, notamment les chlorobenzènes ; des solvants hydrosolubles tels que le diméthylformamide, le diméthylsulfoxyde, la N-méthyl-pyrrolidone ; gaz liquéfiés, etc..).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des

esters d'acides gras et de polyols, les dérivés à fonction sulfates, sulfonates et phosphates des composés précédents. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 0,5 et 80 % pour ces derniers cas).

Comme formes de compositions liquides ou destinées à constituer des compositions liquides lors de l'application, on peut citer les solutions, en particulier les concentrés émulsionnables, les émulsions, les suspensions concentrées, les aérosols, les poudres mouillables (ou poudre à pulvériser), les granulés autodispersibles et les pâtes.

Les concentrés émulsionnables ou solubles comprennent également le plus souvent 10 à 80 % de matière active, les émulsions ou solutions prêtes à l'application contenant, quant à elles, 0,01 à 20 % de matière active. En plus du solvant, les concentrés émulsionnables peuvent contenir, quand c'est nécessaire, 2 à 20 % d'additifs appropriés, comme des stabilisants, des agents tensioactifs, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application

sur les végétaux.

A titre d'exemple, voici la composition de quelques concentrés émulsionnables :

| | |
|---|---|
| - matière active | 250 g |
| - alkylphénol polyéthoxylé | 30 g |
| - alkylarylsulfonate de calcium | 50 g |
| - coupe de distillation du pétrole, distillant entre 160 et 185°C | 670 g |

Autre formule :

| | |
|---|---|
| - matière active | 350 g |
| - huile de ricin polyéthoxylée | 60 g |
| - alkylarylsulfonate de sodium | 40 g |
| - cyclohexanone | 150 g |
| - xylène | 400 g |

Autre formule :

| | |
|---|---|
| - matière active | 400 g |
| - alkyl phénol polyéthoxylé | 100 g |
| - éther méthylique de l'éthylène glycol | 250 g |
| - coupe pétrolière aromatique distillant entre 160-185°C | 250 g |

Autre formule :

| | |
|---|---|
| - matière active | 400 g |
| - phosphate de tristyrylphénol polyéthoxylé | 50 g |
| - phosphate d'alkylphénol polyéthoxylé | 65 g |
| - alkyl benzène sulfonate de sodium | 35 g |
| - cyclohexanone | 300 g |
| - coupe pétrolière aromatique distillant entre 160-185°C | 150 g |

Autre formule :

| | |
|---|---|
| - matière active | 400 g/l |
| - dodécylbenzène sulfonate alcalin | 24 g/l |
| - nonylphénol oxyéthylé à 10 molécules d'oxyde d'éthylène | 16 g/l |
| - cyclohexanone | 200 g/l |
| - solvant aromatique | q.s.p. 1 litre. |

Selon une autre formule de concentré émulsionnable, on

utilise :

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

Les suspensions concentrées, qui sont applicables en pulvérisation, sont préparées de manière à obtenir un produit fluide stable ne se déposant pas (broyage fin) et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu soluble ou non soluble : certaines matières solides organiques ou des sels minéraux peuvent être dissous dans le support pour aider à empêcher la sédimentation ou comme antigels pour l'eau.

A titre d'exemple, voici une composition de suspension concentrée :

| | |
|---|---|
| - matière active | 500 g |
| - phosphate de tristyrylphénol polyéthoxylé | 50 g |
| - alkylphénol polyéthoxylé | 50 g |
| - polycarboxylate de sodium | 20 g |
| - éthylène glycol | 50 g |
| - huile organopolysiloxanique (antimousse) | 1 g |
| - polysaccharide | 1,5 g |
| - eau | 316,5 g |

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quant c'est nécessaire, de 0 à 10 % d'un ou

plusieurs stabilisants et/ou autres additifs, comme des agents de pénétration, des adhésifs, ou des agents antimottants, colorants, etc..

A titre d'exemple, voici diverses compositions de poudres mouillables :

- matière active (composé n°4) 50 %
- lignosulfonate de calcium (défloculant) 5 %
- isopropylnaphtalène sulfonate (agent mouillant anionique) 1 %
- silice antimottante 5 %
- kaolin (charge) 39 %

Un autre exemple de poudre mouillable à 80 % est donné ci-après :

- matière active (composé n°7) 80 %
- alkylnaphtalène sulfonate de sodium 2 %
- lignosulfonate de sodium 2 %
- silice antimottante 3 %
- kaolin 13 %

Un autre exemple de poudre mouillable est donné ci-après :

- matière active (composé n°14) 50 %
- alkylnaphtalène sulfonate de sodium 2 %
- méthyl cellulose de faible viscosité 2 %
- terre de diatomées 46 %

Un autre exemple de poudre mouillable est donné ci-après :

- matière active (composé n°19) 90 %
- dioctylsulfosuccinate de sodium 0,2 %
- silice synthétique 9,8 %

Une autre composition de poudre à pulvériser à 40 % utilise les constituants suivants :

- matière active (composé n°33) 400 g
- lignosulfonate de sodium 50 g
- dibutylnaphtalène sulfonate de sodium 10 g
- silice 540 g

Une autre composition de poudre à pulvériser à 25 %

utilise les constituants suivants :

- matière active (composé n°37) 250 g
- isooctylphénoxy-polyoxyéthylène-éthanol 25 g
- mélange équipondéral de craie de Champagne et d'hydroxyéthylcellulose 17 g
- aluminosilicate de sodium 543 g
- kieselguhr 165 g

Une autre composition de poudre à pulvériser à 10 % utilise les constituants suivants :

- matière active (composé n°42) 100 g
- mélange de sels de sodium de sulfates d'acides gras saturés 30 g
- produit de condensation d'acide naphtalène sulfonique et de formaldéhyde 50 g
- kaolin 820 g

Pour obtenir ces poudres à pulvériser ou poudres mouillables, on mélange intimement les matières actives dans des mélangeurs appropriés avec les substances additionnelles ou on imprègne la matière active fondue sur la charge poreuse et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles de végétaux.

Les granulés "autodispersibles" (en langue anglaise "dry flowable" ; il s'agit plus exactement de granulés facilement dispersibles dans l'eau) ont une composition sensiblement voisine de celle des poudres mouillables. Ils peuvent être préparés par granulation de formulations décrites pour les poudres mouillables, soit par voie humide (mise en contact de la matière active finement divisée avec la charge inerte et avec un peu d'eau, par exemple 1 à 20%, ou de solution aqueuse de dispersant ou de liant, puis séchage et tamisage), soit par voie sèche (compactage puis

broyage et tamisage).

A titre d'exemple, voici une formulation de granulé autodispersible :

| | |
|---|---|
| - matière active | 800 g |
| - alkylnaphtalène sulfonate de sodium | 20 g |
| - méthylène bis naphtalène sulfonate de sodium | 80 g |
| - kaolin | 100 g |

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et elles peuvent avoir une consistance épaisse comme celle d'une "mayonnaise".

Toutes ces dispersions ou émulsions aqueuses ou bouillies sont applicables aux cultures à désherber par tout moyen convenable, principalement par pulvérisation, à des doses qui sont généralement de l'ordre de 100 à 1 200 litres de bouillie à l'hectare.

Les granulés destinés à être disposés sur le sol sont habituellement préparés de manière qu'ils aient des dimensions comprises entre 0,1 et 2 mm et ils peuvent être fabriqués par agglomération ou imprégnation. De préférence, les granulés contiennent 1 à 25 % de matière active et 0 à 10 % d'additifs comme des stabilisants, des agents de modification à libération lente, des liants et des solvants.

Selon un exemple de composition de granulé, on utilise les constituants suivants :

| | |
|---|---|
| - matière active | 50 g |
| - propylène glycol | 25 g |

- huile de lin bouillie 50 g
- argile (granulométrie : 0,3 à 0,8 mm) 910 g

L'invention concerne de plus un procédé de traitement des végétaux contre les champignons phytopathogènes.

Ce procédé est caractérisé en ce qu'il consiste à appliquer sur ces végétaux une quantité efficace d'une composition contenant comme matière acitve un composé selon la formule (I). Par "quantité efficace" on entend une quantité suffisante pour permettre le contrôle et la destruction des champignons présents surs ces végétaux. Les doses d'utilisation peuvent toutefois varier dans de larges limites selon le champignon à combattre, le type de culture, les conditions climatiques, et selon le composé utilisé.

En pratique, on applique avantageusement les matières actives selon l'invention à des doses allant de 10 g/ha à 2 000 g/ha environ, en utilisant pour cela des bouillies contenant la matière active, à la concentration désirée et appliquée à raison de 10 l/ha à 3 000 l/ha.

TABLEAU I

Composés répondant à la formule A

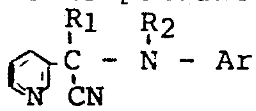

| Composé n° | $R_1$ | $R_2$ | Ar | Point de fusion (°C) | Rendement % |
|---|---|---|---|---|---|
| 1 | $CH_3$ | H | $C_6H_5$ | 156 | 63 |
| 2 | $CH_3$ | H | p-$C_6H_5$-$C_6H_4$ | 189 | 48 |
| 3 | H | $CH_3$ | p-Cl-$C_6H_4$ | 50 | 39 |
| 4 | $CH_3$ | H | p-Cl-$C_6H_4$ | 160 | 21 |
| 5 | $CH_3$ | H | p-F-$C_6H_4$ | 160 | 29 |
| 6 | $CH_3$ | H | o-F-$C_6H_4$ | 140 | 10 |
| 7 | $CH_3$ | H | p-Br-$C_6H_4$ | 157 | 24 |
| 8 | $CH_3$ | H | m,p-$Cl_2$-$C_6H_3$ | 170 | 36 |
| 9 | $CH_3$ | H | m-Cl-$C_6H_4$ | 130 | 22 |
| 10 | $CH_3$ | H | o,p-$Cl_2$-$C_6H_3$ | 168 | 25 |
| 11 | $C_6H_5$ | H | p-F-$C_6H_4$ | 125 | 30 |
| 12 | $C_6H_5$ | H | $C_6H_5$ | 150 | 25 |
| 13 | $CH_3$ | H | o,p-$F_2$-$C_6H_3$ | 133 | 50 |
| 14 | $CH_3$ | H | m-F-$C_6H_4$ | 124 | 41 |
| 15 | $CH_3$ | H | m-Cl,p-F-$C_6H_3$ | 126 | 29 |

TABLEAU I

Composés répondant à la formule A

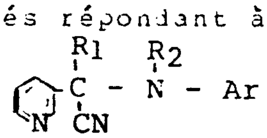

| Composé n° | $R_1$ | $R_2$ | Ar | Point de fusion (°C) | Rendement % |
|---|---|---|---|---|---|
| 16 | $-CH_3$ | H | $m-CN-C_6H_4$ | 164 | 38 |
| 17 | $-CH_3$ | H | $m-Br-C_6H_4$ | 131 | 43 |
| 18 | $-CH_3$ | H | $m-CH_3-C_6H_4$ | 144 | 51 |
| 19 | $-(CH_2)_2CH_3$ | H | $p-Cl-C_6H_4$ | 132 | 21 |
| 20 | $-CH_3$ | H | $m-Br,p-Cl-C_6H_3$ | 173 | 42 |
| 21 | $-(CH_2)_2CH_3$ | H | $m,p-Cl_2C_6H_3$ | 160 | 27 |
| 22 | $-CH_3$ | H | $3,4,5Cl_3-C_6H_2$ | 149 | 10 |
| 23 | $-(CH_2)_2CH_3$ | H | $m-Cl,p-F-C_6H_3$ | 119 | 14 |
| 24 | $-C_6H_5$ | H | $m,p-Cl_2-C_6H_3$ | 180 | 24 |
| 25 | $-CH_2-CH(CH_3)_2$ | H | $p-Cl-C_6H_4$ | 125 | 30 |

TABLEAU I

Composés répondant à la formule A

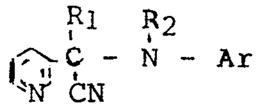

| Composé n° | $R_1$ | $R_2$ | Ar | Point de fusion (°C) | Rendement % |
|---|---|---|---|---|---|
| 26 | $-CH_2-CH(CH_3)_2$ | H | m,p-$Cl_2-C_6H_3$ | 132 | 17 |
| 27 | $-C_6H_5$ | H | m-Br,p-Cl-$C_6H_3$ | 168 | 42 |
| 28 | $-CH_2-CH(CH_3)_2$ | H | m-Cl,p-F-$C_6H_3$ | 113 | 40 |
| 29 | $-CH_3$ | H | p-$(COOCH_3)-C_6H_4$ | 174 | 31 |
| 30 | $-(CH_2)_2CH_3$ | H | p-F-$C_6H_4$ | 116 | 37 |
| 31 | $-CH_3$ | H | m-$SCH_3-C_6H_4$ | 121 | 66 |
| 32 | $-C_2H_5$ | H | p-Cl-$C_6H_4$ | 161 | 49 |
| 33 | $-C_2H_5$ | H | m-Cl-p-F-$C_6H_3$ | 123 | 18 |
| 34 | $-(CH_2)_3-CH_3$ | H | p-Cl-$C_6H_4$ | 114 | 11 |
| 35 | $-C_2H_5$ | H | m,p-$Cl_2-C_6H_3$ | 142 | 61 |
| 36 | $-C_2H_5$ | H | m-Br-$C_6H_4$ | 132 | 11 |
| 37 | $-CH(CH_3)_2$ | H | p-Cl-$C_6H_4$ | 157 | 21 |

TABLEAU I

Composés répondant à la formule A

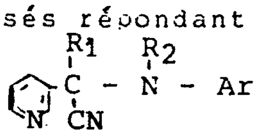

| Composé n° | $R_1$ | $R_2$ | Ar | Point de fusion (°C) | Rendement % |
|---|---|---|---|---|---|
| 38 | $-CH(CH_3)_2$ | H | $p-F-C_6H_4$ | 137 | 43 |
| 39 | $-C_2H_5$ | H | $p-F-C_6H_4$ | 117 | 19 |
| 40 | $-CH_2-C_6H_5$ | H | $m-Cl,p-F-C_6H_3$ | 160 | 55 |
| 41 | $-C_2H_5$ | H | $p-CF_3-C_6H_4$ | 162 | 48 |
| 42 | $-C_2H_5$ | H | $m,p-F_2-C_6H_3$ | 108 | 51 |
| 43 | $-C_2H_5$ | H | $C_6H_5$ | 158 | 75 |
| 44 | $-CH_2-S-CH_3$ | H | $m-Cl,p-F-C_6H_3$ | 119 | 24 |
| 45 | $-(CH_2)_2CH_3$ | H | $p-Br-C_6H_4$ | 146 | 51 |
| 46 | $-C_2H_5$ | H | $p-Br-C_6H_4$ | 176 | 62 |
| 47 | cyclopentyle | H | $p-Cl-C_6H_4$ | 163 | 156 |
| 48 | $-(CH_2)_2CH_3$ | H | $m,p-F_2-C_6H_3$ | 110 | 57 |
| 49 | $-CH(CH_3)_2$ | H | $m,p-F_2-C_6H_3$ | 160 | 28 |
| 50 | $-CH(CH_3)_2$ | H | $p-CF_3-C_6H_4$ | 143 | 19 |

TABLEAU I

Composés répondant à la formule B

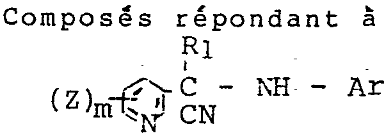

| Composé n° | 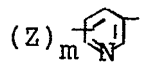 | $R_1$ | Ar | Point de fusion (°C) | Rendement % |
|---|---|---|---|---|---|
| 51 | 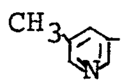 | $-C_2H_5$ | p-Cl-$C_6H_4$ | 147 | 50 |
| 52 | 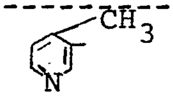 | $-C_2H_5$ | p-Cl-$C_6H_4$ | 146 | 37 |
| 53 | 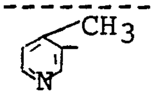 | $-C_2H_5$ | m-Cl-pF-$C_6H_3$ | 155 | 44 |
| 54 | 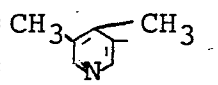 | $-C_2H_5$ | p-Cl-$C_6H_4$ | 123 | 22 |
| 55 | 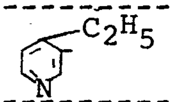 | $-C_2H_5$ | m-Cl,-pF-$C_6H_3$ | 148 | 47 |
| 56 | 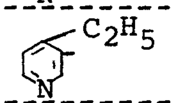 | $-C_2H_5$ | -p-F-$C_6H_5$ H | 117 | 14 |
| 57 | 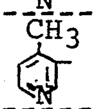 | $CH_3$ | m,p-$Cl_2$-$C_6H_3$ | 156 | 41 |
| 58 | 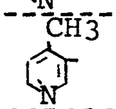 | $CH_3$ | p-Cl-$C_6H_4$ | 180 | 69 |
| 59 | 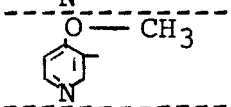 | $-C_2H_5$ | p-Cl-$C_6H_4$ | 89 | 32 |
| 60 | 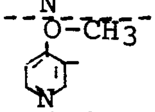 | $CH_3$ | p-Cl-$C_6H_4$ | 171 | 25 |
| 61 | 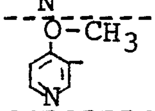 | $CH_3$ | m,p-$Cl_2$-$C_6H_3$ | 145 | 21 |
| 62 | 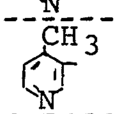 | $-C_2H_5$ | m,p-$F_2$-$C_6H_3$ | 138 | 42 |
| 63 | 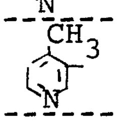 | $-C_2H_5$ | p-Br-$C_6H_4$ | 155 | 43 |
| 72 | 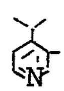 | $-C_2H_5$ | p-F-$C_6H_4$ | 172 | 7 |

## REVENDICATIONS

1) - Dérivé de l'acide (pyridyl-3)-2 phénylamino-2 acétique caractérisé en ce qu'il répond à la formule générale (I)

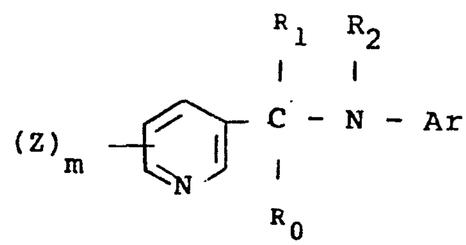

(I)

dans laquelle :

- $R_0$ représente un radical choisi parmi les radicaux -CN, -COOH, $-CONH_2$ et le radical $-COOR_4$ dans lequel $R_4$ représente un radical alkyle inférieur,
- $R_1$ représente l'atome d'hydrogène ou un radical alkyle inférieur, éventuellement substitué ou cycloalkyle inférieur éventuellement substitué, ou phényle éventuellement substitué, ou aralkyle éventuellement substitué,
- $R_2$ représente l'atome d'hydrogène ou un radical alkyle inférieur éventuellement substitué, sous réserve que $R_1$ et $R_2$ ne représentent pas simultanément l'atome d'hydrogène,
- Z représente un atome d'halogène ou un radical alkyle inférieur, alkoxy inférieur ou alkylthio inférieur,
- m est égal à 0, 1, 2, 3 ou 4 étant entendu que lorsque m est supérieur à 1, les substituants Z peuvent être soit identiques, soit différents,
- Ar représente un radical phényle éventuellement substitué,

et sels obtenus par réaction du composé selon la formule (I) avec un acide ou une base approprié,
ainsi que formes stéréoisomères de ce composé, seules ou en mélanges entre elles.

2) - Composé selon la revendication 1, caractérisé en ce

qu'il répond à la formule (II) :

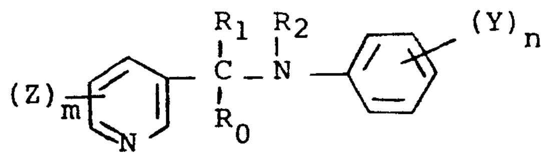 (II)

dans laquelle :

- $R_0$ représente le groupe -CN ou un groupe $-COOR_4$ dans lequel $R_4$ a la même signification que dans la revendication 1,
- $R_1$ représente un radical alkyle inférieur, ou cycloalkyle inférieur,
- $R_2$ représente l'atome d'hydrogène ou le radical méthyle,
- Z représente un radical alkyle ou alkoxy, comportant de 1 à 3 atomes de carbone,
- m est égal à 0, 1 ou 2 étant entendu que lorsque m est égal à 2, les substituants Z peuvent être soit identiques soit différents,
- Y représente un atome d'halogène ou un radical alkyle comportant de 1 à 3 atomes de carbone, halogéno-alkyle comportant de 1 à 3 atomes de carbone, alkoxy ou alkylthio comportant de 1 à 3 atomes de carbone, alkoxycarbonyle inférieur ou phényle, ou cycloalkyle inférieur
- n est égal à 0, 1, 2, 3 ou 4, étant entendu que lorsque n est supérieur à 1, les substituants Y peuvent être soit identiques, soit différents,
- et sels acceptables en agriculture de ces composés.

3) - Composé selon la revendication 2 caractérisé en ce que, dans la formule (II) :

- $R_0$ a la même signification que dans la revendication 2,
- $R_1$ représente un radical alkyle comportant de 1 à 5 atomes de carbone,
- $R_2$ représente l'atome d'hydrogène,
- Z représente un radical méthyle, éthyle ou méthoxy,
- m est égal à 0, 1 ou 2

- Y représente un atome de fluor, chlore ou brome,
- n est égal à 1 ou 2,

étant entendu que, lorsque m est égal à 2, les substituants Z peuvent être soit identiques soit différents, et que lorsque n est égal à 2, les substituants Y peuvent être soit identiques soit différents,

- et sels acceptables en agriculture de ces composés.

4) - Procédé de préparation d'un composé de formule (I) :

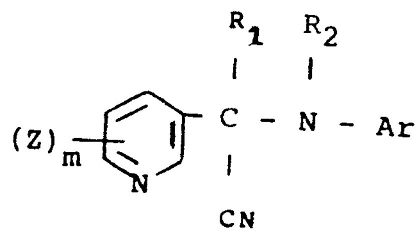

(I)

dans laquelle $R_1$, $R_2$, $R_3$ Ar Z et m ont la même signification que dans la revendication 1, caractérisé en ce qu'il consiste à faire réagir le dérivé de pyridine de formule (III) :

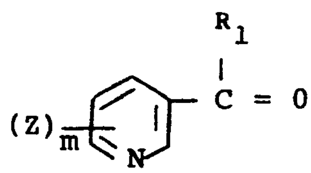

(III)

dans laquelle $R_1$ Z et m ont la même signification que précédemment, sur le dérivé d'aniline de formule (IV) :

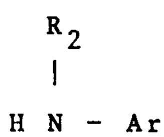

(IV)

dans laquelle Ar et $R_2$ ont la même signification que précédemment, en présence d'acide cyanhydrique éventuellement préparé

"in situ".

5) Procédé de préparation d'un composé de formule (I) :

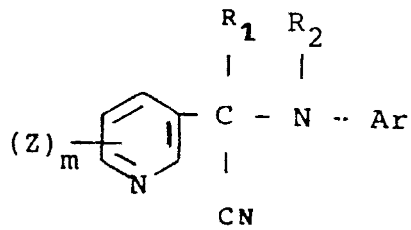

(I)

dans laquelle $R_2$ représente l'atome d'hydrogène et $R_1$, Ar, Z et m ont la même signification que dans la revendication 1, caractérisé en ce qu'il consiste,

- en une première étape, à faire réagir le dérivé de pyridine de formule (III) :

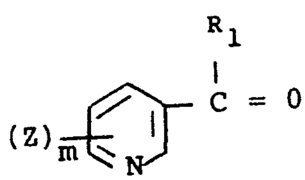

(III)

dans laquelle $R_1$ Z et m ont la même signification que précédemment,

- sur le dérivé d'aniline de formule (V) :

$$H_2 - N - Ar \qquad (V)$$

dans laquelle Ar a la même signification que précédemment,

- pour donner le dérivé de phényliminométhyl-3 pyridine de formule (VI) :

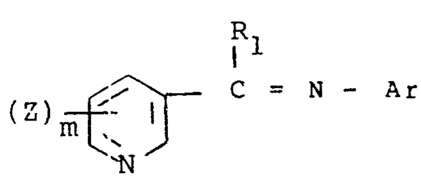

(VI)

dans laquelle $R_1$, Ar, Z et m ont la même signification que précédemment,

- puis en deuxième étape à faire réagir l'acide cyanhydrique, éventuellement formé "in situ", avec ce dérivé de phényliminométhyl-3 pyridine, de formule (VI),

6)- Procédé de préparation d'un composé selon la revendication 1 pour lequel $R_0$ représente le radical $-CO-NH_2$ caractérisé en ce qu'il consiste à hydrater le composé selon la formule (I) pour lequel $R_0$ représente le radical -CN,

7) - Procédé de préparation d'un composé selon la revendication 1 pour lequel $R_0$ représente le radical -COOH caractérisé en ce qu'il consiste à hydrolyser en milieu acide le composé selon la formule I, pour lequel $R_0$ représente le radical $-CONH_2$, puis à neutraliser par une base le composé formé.

8) - Procédé de préparation d'un composé selon la revendication 1, pour lequel $R_0$ représente le radical $-COOR_4$ caractérisé en ce qu'il consiste à estérifier par un alcool de formule $R_4OH$ le composé selon la formule I pour lequel $R_0$ représente le radical -COOH.

9) - Composition antifongique, à usage agricole, caractérisée en ce qu'elle contient comme matière active un composé selon l'une des revendications 1 à 3.

10) - Composition selon la revendication 9, caractérisée en ce que, en plus de la matière active elle comprend un support inerte et/ou un agent tensioactif, utilisable en agriculture.

11) - Composition selon la revendication 10, caractérisée en ce qu'elle comprend de 0,001 % à 95 % en poids de matière active et de 0 à 20 % en poids d'agent tensioactif.

12) - Procédé de traitement des végétaux contre les champignons phytopathogènes caractérisé en ce qu'il consiste à appliquer sur ces végétaux une quantité efficace d'un composé selon l'une des revendications 1 à 3.

## REVENDICATIONS pour l'AUTRICHE

1) - Composition antifongique à usage agricole caractérisée en ce qu'elle contient comme matière active un dérivé de l'acide (pyridyl-3)-2 phénylamino-2 acétique répondant à la formule générale (I)

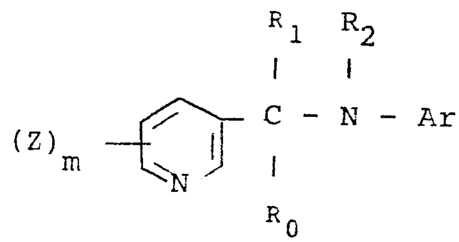

(I)

dans laquelle :

- $R_0$ représente un radical choisi parmi les radicaux -CN, -COOH, $-CONH_2$ et le radical $-COOR_4$ dans lequel $R_4$ représente un radical alkyle inférieur,
- $R_1$ représente l'atome d'hydrogène ou un radical alkyle inférieur, éventuellement substitué ou cycloalkyle inférieur éventuellement substitué, ou phényle éventuellement substitué, ou aralkyle éventuellement substitué,
- $R_2$ représente l'atome d'hydrogène ou un radical alkyle inférieur éventuellement substitué, sous réserve que $R_1$ et $R_2$ ne représentent pas simultanément l'atome d'hydrogène,
- Z représente un atome d'halogène ou un radical alkyle inférieur, alkoxy inférieur ou alkylthio inférieur,
- m est égal à 0, 1, 2, 3 ou 4 étant entendu que lorsque m est supérieur à 1, les substituants Z peuvent être soit identiques, soit différents,
- Ar représente un radical phényle éventuellement substitué,

ou un sel obtenu par réaction de ce composé selon la formule (I) avec un acide ou une base approprié, ou une forme stéréoisomère de ce composé, seule ou en

mélange avec d'autres formes stéréoisomères de ce composé.

2) - Composition selon la revendication 1, caractérisée en ce qu'elle contient comme matière active un composé répondant à la formule :

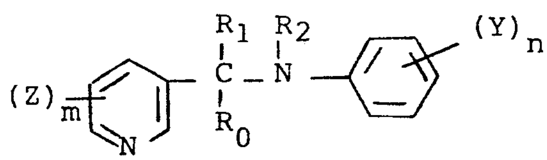

(II)

dans laquelle :

- $R_0$ représente le groupe -CN ou un groupe -$COOR_4$ dans lequel $R_4$ a la même signification que dans la revendication 1,
- $R_1$ représente un radical alkyle inférieur, ou cycloalkyle inférieur,
- $R_2$ représente l'atome d'hydrogène ou le radical méthyle,
- Z représente un radical alkyle ou alkoxy, comportant de 1 à 3 atomes de carbone,
- m est égal à 0, 1 ou 2 étant entendu que lorsque m est égal à 2, les substituants Z peuvent être soient identiques soient différents,
- Y représente un atome d'halogène ou un radical alkyle comportant de 1 à 3 atomes de carbone, halogéno-alkyle comportant de 1 à 3 atomes de carbone, alkoxy ou alkylthio comportant de 1 à 3 atomes de carbone, alkoxycarbonyle inférieur ou phényle, ou cycloalkyle inférieur
- n est égal à 0, 1, 2, 3 ou 4, étant entendu que lorsque n est supérieur à 1, les substituants Y peuvent être soit identiques, soit différents,
- ou un sel acceptable en agriculture de ce composé.

3) - Composition selon la revendication 2 caractérisée en ce que, dans la formule (II) :

- $R_0$ a la même signification que dans la revendication 2,
- $R_1$ représente un radical alkyle comportant de 1 à 5 atomes de carbone,

- $R_2$ représente l'atome d'hydrogène,
- Z représente un radical méthyle, éthyle ou méthoxy,
- m est égal à 0, 1 ou 2
- Y représente un atome de fluor, chlore ou brome,
- n est égal à 1 ou 2,

étant entendu que, lorsque m est égal à 2, les substituants Z peuvent être soit identiques soit différents, et que lorsque n est égal à 2, les substituants Y peuvent être soit identiques soit différents,

- ou un sel acceptable en agriculture de ce composé.

4) - Composition selon l'une des revendications 1 à 3 caractérisée en ce que en plus de la matière active elle comprend un support inerte et/ou un agent tensioactif, utilisable en agriculture.

5) - Composition selon la revendication 4, caractérisée en ce qu'elle comprend de 0,001 % à 95 % en poids de matière active et de 0 à 20 % en poids d'agent tensioactif.

6) - Procédé de traitement des végétaux contre les champignons phytopathogènes caractérisé en ce qu'il consiste à appliquer sur ces végétaux une quantité efficace d'une composition selon l'une des revendications 1 à 5. :

7) - Procédé de préparation d'un composé de formule (I) :

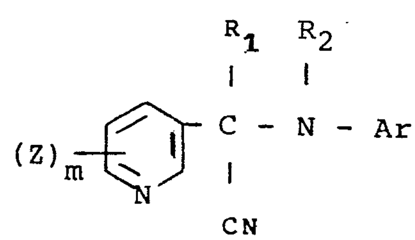

(I)

dans laquelle $R_1$, $R_2$, $R_3$ Ar Z et m ont la même signification que dans la revendication 1, caractérisé en ce qu'il consiste à faire réagir le dérivé de pyridine de formule (III) :

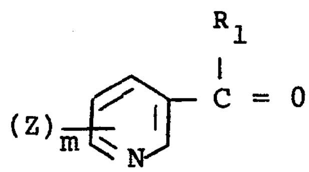

(III)

dans laquelle $R_1$ Z et m ont la même signification que précédemment,

sur le dérivé d'aniline de formule (IV) :

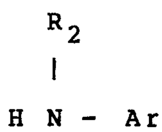

(IV)

dans laquelle Ar et $R_2$ ont la même signification que précédemment,

en présence d'acide cyanhydrique éventuellement préparé "in situ".

8) Procédé de préparation d'un composé de formule (I) :

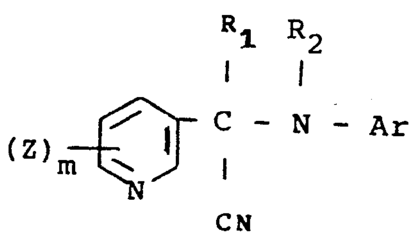

(I)

dans laquelle $R_2$ représente l'atome d'hydrogène et $R_1$, Ar, Z et m ont la même signification que dans la revendication 1, caractérisé en ce qu'il consiste,

- en une première étape, à faire réagir le dérivé de pyridine de formule (III) :

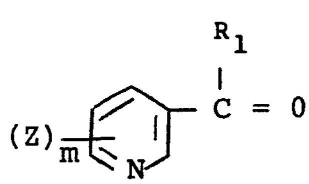

(III)

dans laquelle $R_1$ Z et m ont la même signification que précédemment,

- sur le dérivé d'aniline de formule (V) :

$$H_2 - N - Ar \qquad (V)$$

dans laquelle Ar a la même signification que précédemment,

- pour donner le dérivé de phényliminométhyl-3 pyridine de formule (VI) :

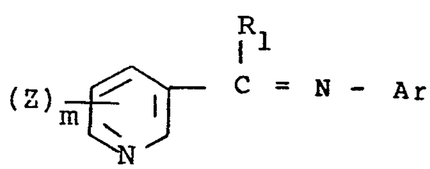

(VI)

dans laquelle $R_1$, Ar, Z et m ont la même signification que précédemment,

- puis en deuxième étape à faire réagir l'acide cyanhydrique, éventuellement formé "in situ", avec ce dérivé de phényliminométhyl-3 pyridine, de formule (VI),

9)- Procédé de préparation d'un composé selon la formule (I) de la revendication 1 pour lequel $R_0$ représente le radical $-CO-NH_2$ caractérisé en ce qu'il consiste à hydrater le composé selon la formule (I) pour lequel $R_0$ représente le radical -CN,

10) - Procédé de préparation d'un composé selon la formule (I) de la revendication 1, pour lequel $R_0$ représente le radical -COOH caractérisé en ce qu'il consiste à hydrolyser en milieu acide le composé selon la formule (I), pour lequel $R_0$ représente le radical $-CONH_2$, puis à neutraliser par une base le composé formé.

11) - Procédé de préparation d'un composé selon la formule (I) de la revendication 1, pour lequel $R_0$ représente le radical $-COOR_4$ caractérisé en ce qu'il consiste à

estérifier par un alcool de formule $R_4OH$ le composé selon la formule I pour lequel $R_0$ représente le radical -COOH.